# EUROPEAN PATENT APPLICATION

(11) **EP 1 847 528 A1**
(43) Date of publication of application: **24.10.2007**
(21) Application number: 06009090.9
(22) Date of filing: 18.04.2006
(51) Int. Cl.: C07C 311/08, C07C 311/11, C07C 307/10, C07C 311/32, C07F 9/24, A61K 8/46, A61K 8/55

(54) **Coupling compounds and hair dyeing compositions containing them**

(71) Applicant: DyStar Textilfarben GmbH & Co. Deutschland KG, 65926 Frankfurt am Main (DE); Kao Corporation, Tokyo 131-8501 (JP)
(72) Inventor: Kühlwein, Jürgen, 63150 Heusenstamm (DE); Russ, Werner, 65439 Flörsheim-Wicker (DE); Pratt, Dominic, 64572 Büttelborn (DE); Möhring, Hartmut, 64342 Seeheim-Jugenheim (DE)
(74) Representative: Muley, Ralf

(57) **Abstract**

The present invention refers to compounds of the formula (I) 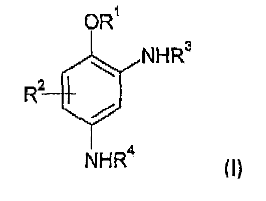 wherein
R¹
is (C₁-C₄)-alkyl, (C₁-C₄)-alkyl which is substituted by hydroxy, (C₁-C₄)-alkoxy, hydroxy-(C₁-C₄)-alkoxy, CN, -COOR⁵, -CON(R⁵)₂ or -N(R⁵)₂ or is phenyl;
R²
is hydrogen, methyl or ethyl;
R³
is (C₁-C₄)-alkylsulfonyl, (C₁-C₄)-alkylsulfonyl which is substituted by hydroxy, halogen, cyano, (C₁-C₄)-alkoxy or -N(R⁵)₂ or is -SO₂-CH=CH₂, -SO₂N(R⁵)₂ or -PO(OR⁵)₂;
R⁴
is hydrogen, (C₂-C₄)-alkyl which is substituted by hydroxy, (C₁-C₄)-alkoxy or hydroxy-(C₁-C₄)-alkoxy;
each of R⁵, independently is hydrogen, (C₁-C₄)-alkyl or hydroxy-(C₂-C₄)-alkyl; whereas R³ is not -NHSO₂CH₃ if R² and R⁴ are both hydrogen and R¹ is methyl, as well as hair dye compostions containing them.

## Description

The present invention relates to novel coupling compounds and hair dye compositions containing them.

It is known to colour human hair with dyeing compositions containing oxidation dye precursors, also called oxidation bases or developers.
Oxidation bases are colourless or weakly coloured compounds, such as ortho- or para-phenylenediamines, ortho- or para-aminophenols or heterocyclic compounds and react with oxidizing agents to give coloured compounds.
The obtained shades can be varied by combining the oxidation bases with couplers, such as aromatic meta-diamines, meta-aminophenols, metahydroxyphenols and certain heterocyclic compounds. A variety of oxidation bases and couplers can be used to enable to get a broad range of different shades.
The permanent colours obtained by using these oxidation dyes need to fulfil some requirements. They should be safe, provide good intensity evenly along the hair shaft, should be stable to external influences, such as shampooing, light, sweat and rubbing and should enable the coverage of grey hair. The dyes should also be stable in the formulation.
There is a constant need to get new developers and couplers that improve at least one of these requirements, particularly there is a constant need to find new couplers which provide improved colour properties
m-Phenylendiamines and their use coupling compounds to produce oxidation dyes for hair dyeing are known and for example described in DE 35 21 995 A1, WO1988/00042, WO1993/010744, DE 102 60 834 A1 and WO2004/058204.

It was now surprisingly found that specific compounds according to the definition given below can be used as couplers and fulfil the needs mentioned above.

The present invention refers to compounds of the formula (I) 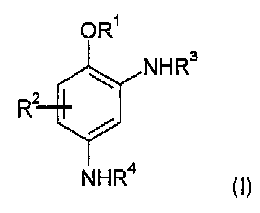 wherein
- R¹: is (C₁-C₄)-alkyl, (C₁-C₄)-alkyl which is substituted by hydroxy, (C₁-C₄)-alkoxy, hydroxy-(C₁-C₄)-alkoxy, CN, -COOR⁵, -CON(R⁵)₂ or -N(R⁵)₂ or is phenyl;
- R²: is hydrogen, methyl or ethyl;
- R³: is (C₁-C₄)-alkylsulfonyl, (C₁-C₄)-alkylsulfonyl which is substituted by hydroxy, halogen, cyano, (C₁-C₄)-alkoxy or -N(R⁵)₂ or is -SO₂-CH=CH₂, -SO₂N(R⁶)₂ or -PO(OR⁵)₂;
- R⁴: is hydrogen, (C₂-C₄)-alkyl which is substituted by hydroxy, (C₁-C₄)-alkoxy or hydroxy-(C₁-C₄)-alkoxy;
each of R⁵, independently is hydrogen, (C₁-C₄)-alkyl or hydroxy-(C₂-C₄)-alkyl;
whereas R³ is not -NHSO₂CH₃ if R² and R⁴ are both hydrogen and R¹ is methyl.

(C₁-C₄)-Alkyl groups may be straight-chain or branched and are for example methyl, ethyl, n-propyl, isopropyl, n-butyl, n-pentyl, isopentyl or n-hexyl. The same logic applies for alkoxy groups, which accordingly are for example methoxy and ethoxy.
Halogen is preferably fluorine, chlorine, bromine and iodine.

The compounds of the formula (I) can be present in form of their acid addition salts of organic or inorganic acids. Examples of such acid additions salts are cosmetically acceptable salts like chlorides, sulfates, phosphates, acetates, propionates, lactates and citrates.

In formula (I) R² is preferably hydrogen and R¹ is preferably not ethyl in case R³ is -NHSO₂CH₃ and R² and R⁴ are both hydrogen.

In especially preferred compounds of the formula (I)
R⁷ is methyl, ethyl, hydroxyethyl, methoxyethyl or -CH₂COOR⁵;
R² is hydrogen;
R³ is methylsulfonyl or ethylsulfonyl which is unsubstituted or substituted by hydroxy, chlorine or -N(R⁵)₂ or is -SO₂-CH = CH₂, -PO(OR⁵)₂ or -SO₂N(R⁵)₂;
R⁴ is hydrogen or methyl or ethyl, which is substituted by hydroxy; and
each of R⁵, independently is hydrogen, methyl, ethyl or hydroxyethyl,
whereas R³ is not -NHSO₂CH₃ if R⁴ is hydrogen and R¹ is methyl.

Examples of especially preferred compounds of formula (I) are the compounds of formulae (Ia) to (Ih)

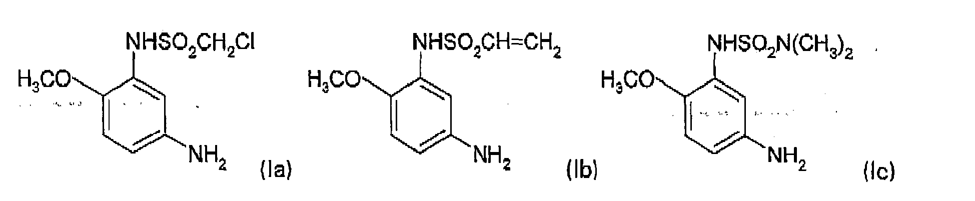

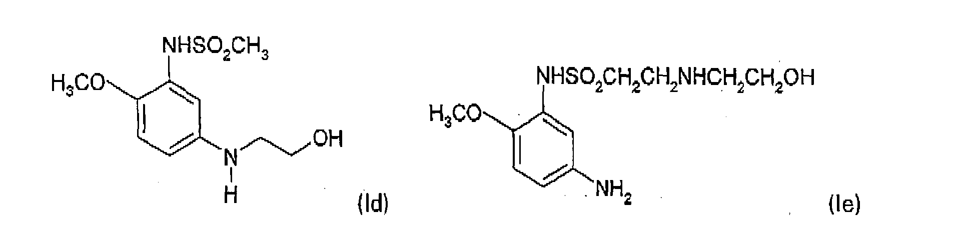

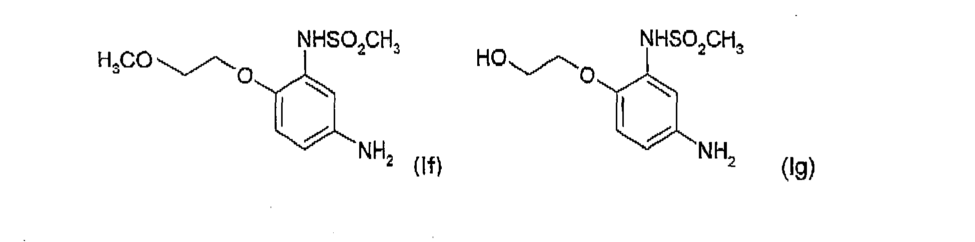

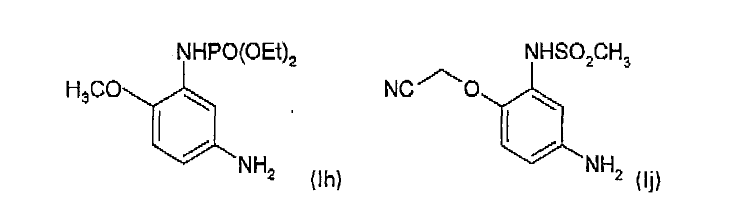

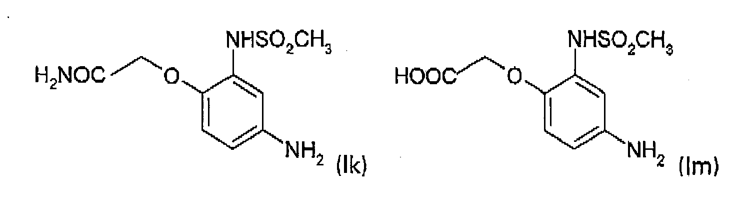

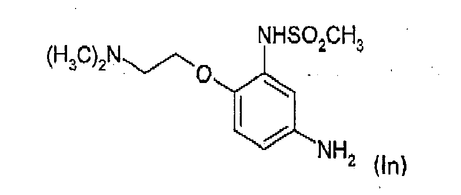

The inventive compounds of formula (I) can be prepared by methods which are in principle known to a person of ordinary skill in the art and which are described in literature, for example in the patent literature cited above.

For example, a compound of the formula (II) 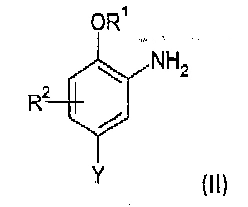 wherein R¹ and R² are defined as given above and Y is a group which can be transferred into an amino group, can be reacted with a compound of the formula (III)

R³-Hal (III)

wherein R³ is defined as given above and Hal is a halogen atom, preferably chlorine, to a compound of the formula (IV) 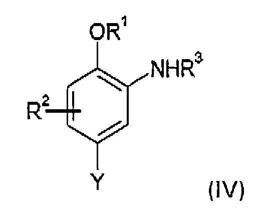 which subsequently can be transferred into a compound of formula (I) wherein R⁴ is hydrogen, which optionally can be further reacted to obtain a compound of formula (I) wherein R⁴ is other than hydrogen.

For example, in case R⁴ is 2-hydroxyethyl the compound of formula (I) wherein R⁴ is hydrogen can be reacted with chloroformic acid 2-chloroethylester followed by a rearrangement to obtain the compound of the formula (lo) 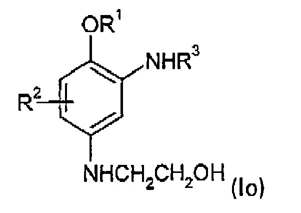 wherein R¹ to R³ are defined as given above.

A group Y which can be transferred into an amino group, with other words which is a protected amino group, is preferably the acetylamino group or the nitro group. The acetylamino group can be deacetylized to form the amino group and the nitro group can be reduced to form the amino group, both being methods known to a skilled person.

The compounds of formula (II) are known and can be obtained by methods known to a person of ordinary skill in the art.
For example, a compound of formula (IIa) 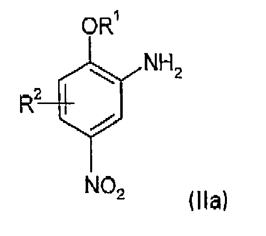 can be obtained by reacting a compound of formula (V) 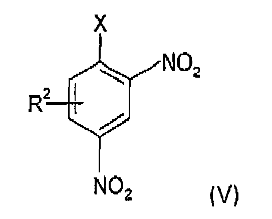 wherein X is fluorine, chlorine or bromine and R² is defined as given above with a compound of the formula (VI)

R¹-OH (VI)

wherein R¹ is defined as given above, and subsequent reduction of the nitro group which is in ortho-position to X.
Alternatively, the compound of formula (IIa) can be obtained by reacting a compound of formula (VII) 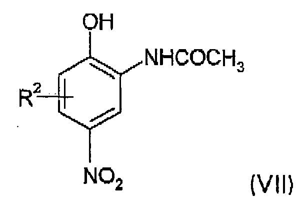 with a compound of formula (VIII)

R¹-Hal (VIII)

wherein R¹ is defined as given above, and subsequent deacetylization of the -NHCOCH₃ group.

The inventive compounds of the formula (I) can advantageously be used as couplers which by reacting with developers form oxidation dyes for hair dyeing, especially human hair dyeing.
Accordingly, the present invention also refers to hair dye compositions comprising at least one compound of the general formula (I).

The inventive hair dye compositions preferably comprise in addition to the compounds of the formula (I) as couplers one or more developers, which couplers and developers are capable to form an oxidation dye for hair dyeing.

Preferred developers are p-phenylenediamine derivatives such as benzene-1,4-diamine (commonly known as p-phenylenediamine), 2-methyl-benzene-1,4-diamine, 2-chloro-benzene-1,4-diamine, N-phenyl-benzene-1,4-diamine, N-(2-ethoxyethyl)benzene-1,4-diamine, 2-[(4-amino-phenyl)-(2-hydroxy-ethyl)-amino]-ethanol (commonly known as N,N-bis(2-hydroxyethyl)-p-phenylenediamine) (2,5-diamino-phenyl)-methanol, 1-(2,5-diamino-phenyl)-ethanol, 2-(2,5-diaminophenyl)-ethanol, N-(4-aminophenyl)benzene-1,4-diamine, 2,6-dimethyl-benzene-1,4-diamine, 2-isopropyl-benzene-1,4-diamine, 1-[(4-aminophenyl)amino]-propan-2-ol, 2-propyl-benzene-1,4-diamine, 1,3-bis[(4-aminophenyl)(2-hydroxyethyl)-amino]propan-2-ol, N⁴,N⁴,2-trimethylbenzene-1,4-diamine, 2-methoxy-benzene-1,4-diamine, 1-(2,5-diaminophenyl)ethane-1,2-diol, 2,3-dimethyl-benzene-1,4-diamine, N-(4-amino-3-hydroxy-phenyl)-acetamide, 2,6-diethylbenzene-1,4-diamine, 2,5-dimethylbenzene-1 ,4-diamine, 2-thisn-2-ylbenzene-1,4-diamine, 2-thien-3-ylbenzene-1,4-diamine, 2-pyridin-3-ylbenzene-1,4-diamine, 1,1'-biphenyl-2,5-diamine, 2-(methoxymethyl)benzene-1,4-diamine, 2-(aminomethyl)benzene-1,4-diamine, 2-(2,5-diaminophenoxy)ethanol, N-[2-(2,5-diaminophenoxy)ethyl]-acetamide, N,N-dimethylbenzene-1,4-diamine, N,N-diethylbenzene-1,4-diamine, N,N-dipropylbenzene-1,4-diamine, 2-[(4-aminophenyl)(ethyl)amino]ethanol, 2-[(4-amino-3-methyl-phonyl)-(2-hydroxy-ethyl)-aminol-ethanol, N-(2-methoxyethyl)-benzene-1,4-diamine, 3-[(4-aminophenyl)amino]propan-1-ol, 3-[(4-aminophenyl)-amino]propane-1,2-diol, N-{4-[(4-aminophenyl)amino]butyl{benzene-1,4-diamine, and 2-[2-(2-}-2-[(2,5-diaminophenyl)-oxy]ethoxy}ethoxy)ethoxy]benzene-1,4-diamine;
p-aminophenol derivatives such as 4-amino-phenol (commonly known as p-aminophenol), 4-methylamino-phenol, 4-amino-3-methyl-phenol, 4-amino-2-hydroxymethyl-phenol, 4-amino-2-methyl-phenol, 4-amino-2-[(2-hydroxy-ethylamino)-methyl]-phenol, 4-amino-2-methoxymethyl-phenol, 5-amino-2-hydroxy-benzoic acid, 1-(5-amino-2-hydroxy-phenyl)-ethane-1,2-diol, 4-amino-2-(2-hydroxy-ethyl)-phenol, 4-amino-3-(hydroxymethyl)phenol, 4-amino-3-fluorophenol, 4-amino-2-(aminomethyl)-phenol, and 4-amino-2-fluoro-phenol; o-aminophenol derivatives such as 2-amino-phenol (commonly known as o-aminophenol), 2,4-diaminophenol, 2-amino-5-methyl-phenol, 2-amino-6-methylphenol, N-(4-amino-3-hydroxy-phonyl)-acetamide, and 2-amino-4-methyl-phenol; and
heterocyclic derivatives such as pyrimidine-2,4,5,6-tetramine (commonly known as 2,4,5,6-tetraaminopyridine), 1-methyl-1H-pyrazole-4,5-diamine, 2-(4,5-diamino-1H-pyrazal-1-yl)ethanol, N<2> N<2> -dimethyl-pyridine-2,5-diamine, 2-[(3-amino-6-methoxypyridin-2-yl)aminolethanol, 6-methoxy-N <2> -methylpyridine-2,3-diamine, 2,5,6-triaminopyrimidin-4(1H)-one, pyridine-2,5-diamine, 1-isopropyl-1H-pyrazole-4,5-diamine, 1-(4-methylbenzyl)-1H-pyrazole-4,5-diamine, 1-(benzyl)-1H-pyrazole-4,5-diamine, 1-(4-chlorobenzyl)-1H-pyrazole-4,5-diamine and 2,5,6-triamino-4-pyrimidinol sulfate.

The hair dye compositions according to the present invention can in addition to the compounds of the formula (I) comprise further couplers. Such further couplers are preferred couplers as normally used to produce oxidation dyes for hair dyeing.
Examples of such couplers are phenols, resorcinol and naphthol derivatives such as naphthalene-1,7-diol, benzene-1,3-diol, 4-chlorobenzene-1,3-diol, naphthalen-1-ol, 2-methyl-naphthalen-1-ol, naphthalene-1,5-diol, naphthalene-2,7-diol, benzene-1,4-diol, 2-methyl-benzene-1,3-diol, 7-amino-4-hydroxy-naphthalene-2-sulfonic acid, 2-isopropyl-5-methylphenol, 1,2,3,4-tetrahydro-naphthalene-1,5-diol, 2-chloro-benzene-1,3-diol, 4-hydroxy-naphthalene-1-sulfonic acid, benzene-1,2,3-triol, naphthalene-2,3-diol, 5-dichloro-2-methylbenzene-1,3-diol, 4,6-dichlorobenzene-1,3-diol, and 2,3-dihydroxy-[1,4]naphthoquinone; m-phenylenediamines such as 2,4-diaminophenol, benzene-1,3-diamine, 2-(2,4-diamino-phenoxy)-ethanol, 2-[(3-amino-phenyl)-(2-hydroxy-ethyl)-aminol-ethanol, 2-mehyl-benzene-1,3-diamlne, 2-[[2-(2,4-diamino-phenoxy)-ethyl]-(2-hydroxyethyl)-amino]-ethanol, 4-{3-[(2,4-diaminophenyl)oxy]propoxy}benzene-1,3-diamine, 2-(2,4-diamino-phenyl)-ethanol, 2-(3-amino-4-methoxy-phenylamino)-ethanol, 4-(2-amino-ethoxy)-benzene-1,3-diamine, (2,4-diamino-phenoxy)-acetic acid, 2-[2,4-diamino-5-(2-hydroxy-ethoxy)-phenoxy]-ethanol, 4-ethoxy-6-methylbenzene-1,3-diamine, 2-(2,4-diamino-5-methyl-phenoxy)-ethanol, 4,6-dimethoxybenzene-1,3-diamine, 2-[3-(2-hydroxy-ethylamino)-2-methyl-phenylamino]-ethanol, 3-(2,4-diamino-phenoxy)-propan-1-ol, N-[3-(dimethylamino)phenyl]urea, 4-methoxy-6-methylbenzene-1,3-diamine, 4-fluoro-6-methylbenzene-1,3-diamine, 2-({3-[(2-hydroxyethyl)amino]-4,6-dimethoxyphenyl}-amino)ethanol, 3-(2,4-diaminophenoxy)-prapane-1,2-diol, 2-[2-amino4-(methylamino)-phenoxy]ethanol, 2-[(5-amino-2-ethoxy-phenyl)-(2-hydroxy-ethyl)-amino]-ethanol, 2-[(3-aminophenyl)amino]ethanol, N-(2-aminoethyl)benzene-1,3-diamine, 4{[(2,4-diamino-phenyl)oxylmethoxy}-benzene-1,3-diamine, and 2,4-dimethoxybenzene-1,3-diamine;
m-aminophenols such as 3-amino-phenol, 2-(3-hydroxy-4-methyl-phenylamino)-acetamide, 2-(3-hydroxy-phenylamino)-acetamide, 5-amino-2-methyl-phenol, 5-(2-hydroxy-ethylamino)-2-methyl-phenol, 5-amino-2,4-dichloro-phenol, 3-amino-2-methyl-phenol, 3-amino-2-chloro-6-methyl-phenol, 5-amino-2-(2-hydroxy-ethoxy)-phenol, 2-chloro-5-(2,2,2-trifluoro-ethylamino)-phenol, 5-amino-4-chloro-2-methyl-phenol, 3-cyclopentylamino-phenol, 5-[(2-hydroxyethyl)amino]4-methoxy-2-methylphenol, 5-amino-4-methoxy-2-methylphenol, 3-(dimethylamino)phenol, 3-(diethylamino)phenol, 5-amino4-fluoro-2-methylphenol, 5-amino-4-ethoxy-2-methylphenol, 3-amino-2,4-dichloro-phenol, 3-[(2-methoxyethyl)amino]phenol, 3-[(2-hydroxyethyl)amino]phenol, 5-amino-2-ethylphenol, 5-amino-2-methoxyphenol, 5-[(3-hydroxypropyl)amino]-2-methylphenol, 3-[(3-hydroxy-2-methylphenyl)-amino]propane-1,2-diol, and 3-[(2-hydroxyethyl)amino]-2-methylphenol; and
heterocyclic derivatives such as: 3,4-dihydro-2H-1,4-benzoxazin-6-ol, 4-methyl-2-phenyl-2,4-dihydro-3H-pyrazol-3-one, 6-methoxyquinolin-8-amine, 4-methylpyridine-2,6-diol, 2,3-dihydro-1,4-benzodioxin-5-ol, 1,3-benzodioxol-5-ol, 2-(1,3-benzodioxol-5-ylamino)ethanol, 3,4-dimethylpyridine-2,6-diol, 5-chloropyridine-2,3-diol, 2,6-dimethoxypyridine-3,5-diamine, 1,3-benzodiaxal-5-amine, 2-{[3,5-diamino-6-(2-hydroxy-ethoxy)-pyridin-2-yl]oxy}-ethanol, 1H-indol-4-ol, 5-amino-2,6-dimethoxypyridin-3-ol, 1H-indole-5,6-diol, 1H-indol-7-ol, 1 H-indol-5-ol, 1H-indol-6-ol, 6-bromo-1,3-benzodioxol-5-ol, 2-aminapyridin-3-ol, pyridine-2,6-diamine, 3-[(3,5-diaminopyridin-2-yl)oxy]propane-1,2-diol, 5-[(3,5-diaminopyridin-2-yl)oxy]pentane-1,3-diol, 1H-indole-2,3-dione, indoline-5,6-diol, 3,5-dimethoxypyridine-2,6-diamine, 6-methoxypyridine-2,3-diamine, and 3,4-dihydro-2H-1,4-benzoxazin-6-amine,

The inventive hair dye compositions usually contain couplers at a total concentration of 0.001 to 10%, preferably 0.005 to 7.5% and more preferably 0.01 to 5% by weight, based on the weight of the hair dyeing composition and developers at a total concentration of 0.001 to 10%, preferably 0.005 to 7.5% and more preferably 0.01 to 5% by weight, calculated based on the weight of the hair dyeing composition.

The hair dye compositions according to the present invention can additionally comprise at least one direct dye. Such direct dyes can be cationic, anionic or neutral and can be of natural or synthetic origin. Preferred natural dyes are plant dyestuffs. Suitable dyes of such types are known on the market for hair colouring applications.

Suitable cationic dyestuffs are for example Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57, Basic Orange 31 and Basic Yellow 87.
Additional examples are the dyes disclosed in WO 95/15144 which are included herein by reference.
The hair dye compositions according to the present invention can contain cationic dyestuffs at a concentration of 0.001 to 2%, preferably 0.005 to 1.5% and more preferably 0.01 to 1% by weight, based on the weight of the hair dyeing composition.

Suitable anionic dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium and potassium. The hair dye compositions according to the present invention can contain anionic dyestuffs at a concentration of 0.001 to 2%, preferably 0.005 to 1.5% and more preferably 0.01 to 1 % by weight, based on the weight of the hair dyeing composition.

Suitable neutral dyes (HC dyes or nitro dyes) are HC Blue No.2, HC Blue No.4, HC Blue No.5, HC Blue No.6, HC Blue No.7, HC Blue No.8, HC Blue No.9, HC Blue No.10, HC Blue No. 11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.
The hair dye compositions according to the present invention can contain neutral dyestuffs at a concentration of 0.001 to 2%, preferably 0.01 to 1.5% and more preferably 0.05 to 1 % by weight, based on the weight of the hair dyeing composition.

Suitable plant dyestuffs are henna (red or black), alkanna root, laccaic acid, indigo, logwood powder, madder root and rhubarb powder.
The pH of the hair dye compositions according to the present invention may range from 3 to 12, preferably from 5 to 11. The pH might be adjusted to the desirable value by the use of acidifying and alkalising agents. Suitable acidifying agents are for example mineral and organic acids, such as hydrochloric acid, orthophosphoric acid, sulfuric acid, carboxylic acids such as acetic acid, tartaric acid, citric acid, and lactic acid, and sulphonic acids. Suitable alkalizing agents are for example aqueous ammonia, alkali metal carbonates, alkalonamine such as monoethanolamine, diethanolamine, and triethanolamine, and derivatives thereof, sodium hydroxide.

In addition to the above-described components, those ordinarily employed as a raw material for cosmetics can be added to the hair dye compositions of the present invention. Examples of such an optional component include hydrocarbons such as squalane; animal or vegetable fats and oils; higher fatty acids such as oleic acid; organic solvents such as 1,2-propylene glycol; penetration promoters; cationic surfactants; natural or synthetic polymers; higher alcohols such as cetearyl alcohol; ethers; amphoteric surfactants; nonionic surfactants such as stearoyl monoethanolamide, coconut monoethanolamide, propylene glycol monostearate, polyoxyethylene (5) coconut amide, anionic surfactants such as sodium cetyl sulfate, sodium stearyl sulfate, potassium stearate, sodium stearate, polyoxyethylene (5) oleyl ether phosphate; protein derivatives; active ingredients such as coenzyme Q10; amino acids; antiseptics; chelating agents; stabilizers; antioxidants; plant extracts; crude drug extracts; vitamins such as retinyl palmitate; colorants; perfumes; catalysts such as potassium iodide; and ultraviolet absorbers.

The hair dye compositions of the present invention can be prepared in a conventional manner by mixing the components in the required amounts.

In order to use the hair dye compositions of the present invention to dye hair they have to be mixed with an oxidation agent, which allows the formation of an oxidation dye by reaction of the coupler component with the developer component and which is usually called developer composition. Preferred oxidation agents are peroxide containing agents, particularily hydrogen peroxide, or precursors thereof. Also suitable are urea peroxide, sodium perborate, sodium percarbonate, melamine peroxide, and persulfates such as ammonium persulfate, sodium persulfate and potassium persulfate. Oxygen can also be used as oxidation agent.
Typically, hydrogen peroxide or its addition compounds with urea, melamine, sodium borate or sodium carbonate are used in the form of a 1 % to 20%, preferably 2% to 15 %, most preferably 2% to 12% preparation.

The hair dye composition according to the present invention as well as the oxidation agent can be present in the form of powder, transparent liquid, emulsion, cream, gel, paste, aerosol, aerosol foam or the like.

The present application also refers to a hair dyeing kit which comprises in separate containers a hair dye composition according to the present invention, a developer composition and optionally further components.
Such further components are for example a shampoo, a conditioning composition, a hair treatment product, gloves and instructions for use.
Usually, the hair dye composition according to the present invention and the developer composition (oxidation agent) are mixed just before hair dyeing and the mixture is applied to the hair in a sufficient amount, which depends on the hair abundance, generally from about 60 to 200 grams. The mixture is then allowed to act on the hair for about 10 to about 45 minutes, preferably about 30 minutes, at about 15 to 50°C. Thereafter, the hair is rinsed with water and dried. If necessary, it is washed with a shampoo. Subsequently the hair is dried.

The hair dye composition according to the present invention provides hair dyeing which has good uptake, good selectivity, as well as good stability to light and shampooing with improved brilliance.

### Example 1: N-(5-Amino-2-methoxyphenyl)-chloromethansulfonamide (compound of formula (Ia))

9,0 g of N-(3-Amino-4-methoxyphenyl)-acetamide were suspended in 50 ml of acetonitrile, After addition of 4,4 ml of pyridine 5 ml of chloromethylsulfonyl-chloride, dissolved in 10 ml of acetonitrile, were introduced dropwise. Temperature of the reaction mixture raised to 40-50°C. After addition of sulfonylchloride, temperature was kept at 60°C until no N-(3-Amino-4-methoxyphenyl)-acetamide was detectable. Solvent was removed by evaporation, the residue was taken up with water and undissolved material was isolated by filtration.
This residue was heated without further purification in 50 ml of HCl, 15 % to 90°C for one hour. After cooling the crystallized material was isolated by filtration as hydrochloride.
Yield: 9,7 g (67,5 %)

Following the above described procedure N-(3-Amino-4-methoxyphenyl)-acetamide was converted to further sulfonamides:

### Example 2: Ethenesulfonic acid (5-Amino-2-methoxyphenyl)-amide (compound of formula (Ib)) as hydrochloride

6,3 ml of 2-chloro-1-ethanesulfonylchloride (instead of chloromethylsulfonyl-chloride) and 9,7 ml of pyridine were used, following addition of sulfonyl-chloride at 0-5°C and further reaction at 20°C. The dryed intermediate was deacetylated with hydrogen chloride in methanol (5 moles HCl /I methanol) at 60-65°C. the product obtained was isolated after deacetylation by evaporation to dryness. Yield: 9,9 g (75,1 %)

### Example 3; N-(5-Amino-2-methoxyphenyl)-dimethylsulfamoyl amide (compound of the formula (Ic)

Use of 5,8 ml of dimethylsulfamoylchloride resulted in 7,8 g of acetylated intermediate (54,1 %). Deacetylation was performed in diluted sulphuric acid, 30 % within 1 hour at 100°C. After neutralization the product was isolated by filtration. Yield: 5,6 g (82,2%)

### Example 4: N-(5-(2-Hydroxyethylamino)-2-methoxyphenyl)-methane sulfonamide (compound of formula (Id))

7,25 g of N-(5-amino-2-methoxyphenyl)-methanesulfonamide x HCl was dissolved in a mixture of 40 ml of 1,2- dimethoxyethane and 5 ml of water. After addition of 2,0 g of calciumcarbonate temperature was raised to 80°C. 3,7 ml of chloroformic acid 2-chloroethylester was added drop-wise and thereafter temperature was kept at 80°C for 1 hour. The reaction mixture was diluted with 200 ml water and the carbamate intermediate was isolated by filtration, yielding 24,4 g wet cake.
The wet cake was suspended in a mixture of 60 ml of water and 20 ml of ethanol and thereafter 11,5 g of potassium hydroxide solution, 50 %, was added with a dropping funnel. The reaction mixture was heated to 60°C and kept there for 1 hour. After cooling the mixture was neutralized with acetic acid and the separated material was isolated by filtration.
Yield: 6,9 g (77,5%)

### Example 5: 2-(2-Hydroxyothylemino)-ethansulfonic acid (5-amino-2-methoxyphenyl)-amide (compound of formula (le))

1, 13 g of N-(3-ethenesulfonylamino-4-methoxyphenyl)-acetamide (intermediate of example 2) was dissolved in 10 ml of acetonitrile, 0,91 ml of ethanolamine and a trace copper powder was added. The reaction mixture was heated for 4 hours to 80°C. Thereafter the reaction mixture was filtered and evaporated to dryness. After addition of 15 ml of methanolic hydrogen chloride (5 moles HCl/l methanol) the solution was heated for 2 hours to 60-65°C. The product was isolated as oil after evaporation to dryness.
Yield: 1,75 g (96,3%)

### Example 6: N-(5-Amino-2-(2-methoxyethoxy)phenyl)-methanesulfonamide (compound of formula If))

### a) 2-(2-Methoxyethoxy)-5-nitrophenylamine

48 ml of methylglycol was mixed with 81 g of 2,4-dinitrochlorobenzene. 17,2 g of solid sodium hydroxyde was added in portions in such a manner, that reaction temperature didn't exceed 35°C. Stirring was continued until all sodium hydroxide has dissolved. After dilution with 100 ml of water a solution of 88,6 g of NaHS x H2O was added dropwise between 70 and 80°C. The reaction mixture was kept at 80°C until no dinitro intermediate was detectable. After cooling the crystallized product was isolated by filtration and dried.
Yield: 72,5 g (85,4%)

### b) N-(2-(2-Methoxyethoxy)-5-nitrophenyl)-methanesulfonamide

4,24 g of 2-(2-methoxyethoxy)-5-nitrophenylamine was suspended in 25 ml of acetonitrile. After addition of 2,01 ml pyridine 1,94 ml of methylsulfonylchloride, dissolved in 5 ml of acetonitrile, was introduced dropwise. After addition of sulfonylchloride, the reaction mixture was heated to 60°C and kept there for 4 hours. Solvent was removed by evaporation and the residue was taken up with water; the product was isolated by filtration and dried.
Yield: 5,29 g (91,2 %)

### c) N-(5-amino-2-(2-methoxyethoxy)phenyl)-methanesulfonamide

5,0 g of N-(2-(2-methoxyethoxy)-5-nitrophenyl)-methanesulfonamide was dissolved in 300 ml ethanol. After addition of 0,5 g of Pd/C the material was hydrogenated at 60°C under 50 bar for 1 hour. The reaction solution was filtered and evaporated to dryness; the viscos residue was dissolved in methanolic hydrogen chloride. The crystallized product was isolated by filtration and dried. Yield: 3,72 g (72,8%)

### Example 7: N-(5-Amino-2-(2-hydroxyethoxy)phenyl)-methanesulfonamide (compound of formula Ig))

### a) 2-(2-Amino-4-nitro-phenoxy)-ethanol

40,5 g of 2,4-dinitrochlorobenzene were dissolved in 120 ml of ethylenglycole and heated to 115°C. Then 10,6 g of sodium carbonate were added and heating was continued until conversion was completed. Now ethylengycole was removed by distillation under reduced pressure. The remaining liquid was diluted with 70 ml of water and a solution of 17,6 g NaHS x H₂O in 30 ml water was added dropwise between 70 and 80°C: The reaction mixture was kept at 80°C until no dinitro intermediate was detectable. After addition of 350 g ice the crystallized product was isolated by filtration. Purification of the crude product was done by dissolution in diluted HCl and filtration; after neutralization the crystallized product was recovered by filtration and dried,
Yield: 24,5 g (61,9%)

### b) N-(2-(2-Hydroxyethoxy)-5-nitrophenyl)-methanesulfonamide

3,96 g of 2-(2-amino-4-nitro-phenoxy)-ethanol were suspended in 25 ml of acetonitrile. After addition of 2,01 ml of pyridine 1,94 ml of methylsulfonylchloride, dissolved in 5 ml of acetonitrile, was introduced dropwise. After addition of sulfonylchloride, the reaction mixture was heated to 60°C and kept there for 8 hours. Solvent was removed by evaporation and the residue was taken up with water.The product was isolated by filtration and dried.
Yield: 3,72 g (67,4 %)

### c) N-(5-Amino-2-(2-hydoxyethoxy)phenyl)-methanesulfonamide

3,72 g of N-(2-(2-hydroxyethoxy)-5-nitrophenyl)-methanesulfonamide were dissolved in 300 ml of ethanol. After addition of 0,5 g of Pd/C the material was hydrogenated at 60°C under 50 bar for 1 hour. The reaction solution was filtered and evaporated to dryness and the viscose residue was dissolved in methanolic hydrogen chloride. The crystallized product was isolated by filtration and dried. Yield: 2,90 g (76,2%)

### Example 8: (6-Amino-2-methoxyphonyl)-phosphoramidic acid diethylester (compound of formula (1h))

### a) (2-Methoxy-5-nitrophenyl)-phosphoramidic acid diethylester

8,41 g of 2-methoxy-5-nitrophenylamine was suspended in 50 ml of acetonitrile. After addition of 4,84 ml of pyridine 8,67 ml of diethylchlorophosphate, dissolved in 15 ml of acetonitrile, was introduced dropwise. Temperature of the reaction mixture raised to 40°C. After addition of chlorophosphate, temperature was raised to 60°C and kept there until no 2-methoxy-5-nitrophenyl-amine was detectable. Solvent was removed by evaporation and the residue was taken up with water; the undissolved product was isolated by filtration.
Yield: 14,04 g (92,3%)

### b) (5-Amino-2-methoxyphenyl)-phosphoremidic acid diethylester

48,78 g of iron-II-sulfate heptahydrate was dissolved in 250 ml water and heated under nitrogen to 80°C. 7,61 g of (2-methoxy-5-nitrophenyl)-phosphoramidic acid diethylester were dissolved in 50 ml of hot ethanol and introduced in the hot iron-sulfate solution. Within 30 minutes 63,3 ml of ammonium hydroxide, 26% was added portionwise and the reaction temperature was kept between 85 and 90°C for 1 hour. Then the hot reaction mixture was filtered and after cooling extracted with n-butanol. After drying of the organic layer and evaporation of the solvent the product was received as solid material.
Yield: 6,5 g (94,8%)

### Example 9: (4-Amino-2-methanesulfonylamino-phenoxy)-acetic acid (compound of formula (lm))

### a) (2-Acetylamino-4-nitro-phenoxy)-acetic acid ethylester

A mixture of 9,81g of 2-Acetylamino-4-nitro-phenol, 6,91 g of potassium carbonate and 4,6 g of potassium iodide was suspended in 100 ml acetone. After addition of 6,75 g of chloroacetic acidethylester temperature was raised to 60°C and kept there until no 2-Acetylamino-4-nitro-phenol was detectable. Solvent was removed by evaporation and the residue was taken up with water; the undissolved product was isolated by filtration and dried.
Yield: 13,4 g (94,9%)

### b) (2-Amino-4-nitro-phenoxy)-acetic acid

13,4 g of (2-Acetylamino-4-nitro-phenoxy)-acetic acid ethylester was suspended in 60 ml NaOH, 10 % and heated for 1 hour to 60°C. After cooling pH 4,5 was set with diluted HCl. The separated product was isolated by filtration and dried. Yield; 9,76 g (96,9%)

### c) (2-Methanesulfonylamino-4-nitro-phenoxy)-acetic acid

The procedure described in example 6b) was performed with 9,76 g of (2-Amino-4-nitro-phenoxy)-acetic acid, 5,80 g of methanesulfonylchloride and 4,0 g of pyridine in acetonitrile.
Yield: 11,32 g (84,8%)

### d) (4-Amino-2-methanesulfonylamina-phenoxy)-acetic acid

11,32 g of (2-Methanesulfonylamino-4-nitro-phenoxy)-acetic acid were dissolved in 300 ml of a ethanole water mixture (1:1). After addition of 0,5 g Pd/C the material was hydrogenated at 60°C under 50 bar for 1 hour. The reaction solution was filtered and evaporated to dryness.
Yield: 9,76 g (96,2%)

### Example 10: 2-(4-Amino-2-methanesulfonylamino-phenoxy)-acetamid (compound of formula (Ik))

The procedure of example 9 was repeated with the exception that 5,14 g of chloroacetamide were used instead of 6,75 g of chloroacetic acidethylester.
Yield: 8,3 g (64% over all steps)

### Example 11: N-(5-Amino-2-cyanomethoxy-phenyl)-methanesulfanamide (compound of formula (Ij))

The procedure of example 9 was repeated with the exception that 4,15 g of chloroacetonitrile were used instead of 6,75g of chloroacetic acidethylester.
Yield: 7,72 g (63,9% over all steps)

### Example 12: N-(5-Amino-2-(2-dimethylaminoethoxy)-phenyl)-methanesulfonamide (compound of formula (In))

The procedure of example 9 was repeated with the exception that 7,92 g of dimethylaminoethylchloride hydrochloride were used instead of 6,75 g of chloroacetic acidethylester.
Yield: 6,29 g (46,0% over all steps)

### Application Example

The following Dye Compositions were produced:

| | |
|---|---|
| iso-propanol | 5.0 % |
| ammonia 25% active | 5.0 % |
| sodium sulfite | 1.0 % |
| hydrogen peroxide | 3.0 % |
| developer (I to VI; see below) | 0.4 % |
| coupler (inventive compound; see below) | 0.4 % |
| water | up to 100 % |

The following developers were used:

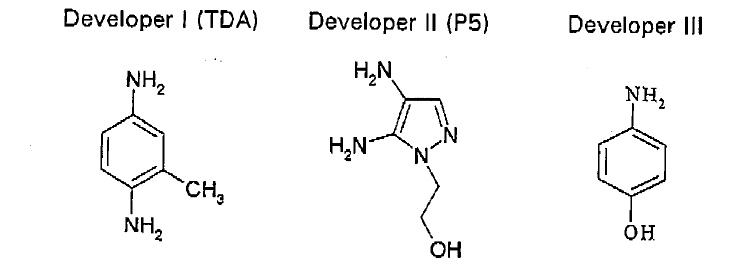

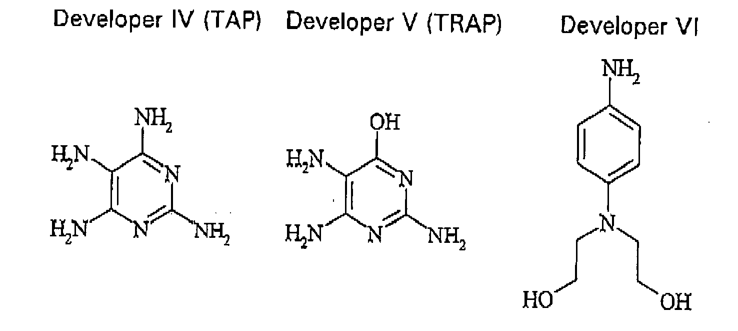

The Dye Compositions mentioned above were applied to undamaged white goat hair in amounts of approximately 1 g per g of hair at 50°C for 15min. or at 30°C for 30min. At the end of the processing time the tresses were rinsed with water, shampooed and then dried.

Brilliant colourings as given in the following Tables 1 and 2 were obtained.

**Table 1**

| Compound of the formula (I) | Developer I | Developer II |
|---|---|---|
| la | brown | intense violet |
| lb | brown | blueish red |
| Ie | light brown | ash |
| Ic | brown | intense violet |
| Ih | brown | intense violet |
| Id | light brown | intense pink |
| lf | brown | intense violet |
| lg | brown | intense violet |
| Ij | light brown | violet |
| Ik | light brown | violet |
| Im | dark brown | intense violet |
| In | brown | violet |

**Table 2**

| Developer | Compound of the formula (Ia) | Compound of the formula (If) |
|---|---|---|
| I | Red brown | brown |
| II | Intense violett | Intense violet |
| III | Orange | Brown |
| IV | Green | Green |
| V | Brownish | Yellowish |
| VI | brown | Red brown |

## Claims

1. Compound of the formula (I) 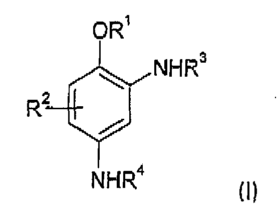 wherein
R¹ is (C₁-C₄)-alkyl, (C₁-C₄)-alkyl which is substituted by hydroxy, (C₁-C₄)-alkoxy, hydroxy-(C₁-C₄)-alkoxy, CN, -COOR⁵, -CON(R⁵)₂ or -N(R⁵)₂ or is phenyl;
R² is hydrogen, methyl or ethyl;
R³ is (C₁-C₄)-alkylsulfonyl, (C₁-C₄)-alkylsulfonyl which is substituted by hydroxy, halogen, cyano, (C₁-C₄)-alkoxy or -N(R⁵)₂ or is -SO₂-CH = CH₂, -SO₂N(R⁵)₂ or -PO(OR⁵)₂;
R⁴ is hydrogen, (C₂-C₄)-alkyl which is substituted by hydroxy, (C₁-C₄)-alkoxy or hydroxy-(C₁-C₄)-alkoxy;
each of R⁵, independently is hydrogen, (C₁-C₄)-alkyl or hydroxy-(C₂-C₄)-alkyl;
whereas R³ is not -NHSO₂CH₃ if R² and R⁴ are both hydrogen and R¹ is methyl.

2. Compound according to claim 1, wherein R² is hydrogen.

3. Compound according to claim 1 and/or 2, wherein
R¹ is methyl, ethyl, hydroxyethyl, methoxyethyl or -CH₂COOR⁵;
R² is hydrogen;
R³ is methylsulfonyl or ethylsulfonyl which is unsubstituted or substituted by hydroxy, chlorine or -N(R⁵)₂ or is -SO₂-CH = CH₂, -PO(OR⁵)₂ or -SO₂N(R⁵)₂; R⁴ is hydrogen or methyl or ethyl, which is substituted by hydroxy; and each of R⁵, independently is hydrogen, methyl, ethyl or hydroxyethyl,
whereas R³ is not -NHSO₂CH₃ if R⁴ is hydrogen and R¹ is methyl.

4. Process for the preparation of a compound of formula (I) according to one or more of claims 1 to 3, wherein a compound of formula (II) 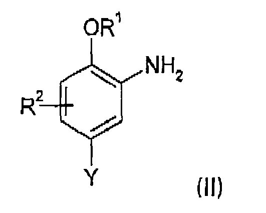 wherein R¹ and R² are defined as given above and Y is a group which can be transferred into an amino group, is reacted with a compound of the formula (III)
R³-Hal (III)
wherein R³ is defined as given above and Hal is a halogen atom, preferably chlorine, to a compound of the formula (IV) 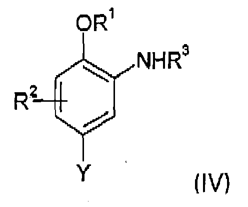 which subsequently is transferred into a compound of formula (I) wherein R⁴ is hydrogen, which optionally is further reacted to obtain a compound of formula (I) wherein R⁴ is other than hydrogen.

5. Hair dye composition comprising one or more compounds of the formula (I) according to one or more of claims 1 to 3.

6. Hair dye composition according to claim 5, which comprises in addition to the compounds of the formula (I) as couplers one or more developers, which couplers and developers are capable to form an oxidation dye for hair dyeing.

7. Hair dye composition according to claims 5 and/or 6, which comprises in addition to the compounds of the formula (I) further couplers.

8. Hair dye composition according to one or more of claims 5 to 7, which comprises at least one direct dye.

9. Hair dyeing kit which comprises in separate containers a hair dye composition according to one or more of claims 5 to 8, a developer composition and optionally further components.
